Europäisches Patentamt

European Patent Office

Office européen des brevets

(19)

(11) Numéro de publication: **0 285 795 B1**

(12) **FASCICULE DE BREVET EUROPEEN**

(45) Date de publication de fascicule du brevet:
22.05.91

(51) Int. Cl.⁵: **A61L 2/04, A61L 2/06**

(21) Numéro de dépôt: 88102881.5

(22) Date de dépôt: 26.02.88

(54) **Procédé de stérilisation d'une suspension aqueuse d'un sel insoluble dans l'eau.**

(30) Priorité: 31.03.87 CH 1228/87

(43) Date de publication de la demande:
12.10.88 Bulletin 88/41

(45) Mention de la délivrance du brevet:
22.05.91 Bulletin 91/21

(84) Etats contractants désignés:
AT BE CH DE ES FR GB GR IT LI LU NL SE

(56) Documents cités:
US-A- 4 219 583
US-A- 4 303 691
US-A- 4 514 433

(73) Titulaire: **SOCIETE DES PRODUITS NESTLE S.A.**
**Case postale 353**
**CH-1800 Vevey(CH)**

(72) Inventeur: **Hugelshofer, Willy**
**Alpenstrasse 15**
**CH-3510 Konolfingen(CH)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 285 795 B1

## Description

L'invention concerne un procédé de stérilisation d'une suspension aqueuse d'un sel ou de sels insolubles dans l'eau pour utilisation ultérieure dans un remplissage aseptique. L'invention concerne en outre l'utilisation de cette suspension stérilisée.

Le remplissage aseptique est connu et permet de préparer des produits ayant une durée de conservation pouvant aller jusqu'à six mois ou plus. C'est le cas notamment du lait. Pour augmenter les qualités nutritionnelles des produits en emballage aseptique, il serait intéressant d'y ajouter des sels minéraux. Or, nombre des sels minéraux potentiellement utilisables sont insolubles dans l'eau. La solution consistant à ajouter directement ces sels minéraux dans le produit, et à stériliser ensuite le mélange obtenu n'est pas possible, car il conduit à un produit final dégradé, en raison de réactions secondaires lors de la stérilisation. Il faut donc envisager une solution de stérilisation séparée du produit de base et des sels minéraux à ajouter. Or, une stérilisation continue d'une suspension aqueuse d'un sel insoluble est pratiquement impossible, en raison de la forte sédimentation du sel, du revêtement des surfaces de chauffage et de refroidissement et de l'abrasion des organes de pompage.

Le procédé, objet de la présente invention, permet d'effectuer une stérilisation d'une suspension aqueuse d'un sel insoluble sans les inconvénients précités. L'invention concerne un procédé de stérilisation d'une suspension aqueuse d'un sel ou de sels insolubles dans l'eau pour utilisation ultérieure dans un remplissage aseptique, dans lequel on ajoute à ladite suspension un composé choisi dans le groupe constitué par la gomme de xanthane et la carboxyméthylcellulose et on la chauffe à une température comprise entre 120 et 150° C.

La gomme de xanthane et la carboxyméthylcellulose sont des épaississants, qui améliorent l'homogénéité de la suspension et sa stabilité pendant le traitement thermique, sans aucune influence sur le produit final stérilisé.

Le sel ou les sels insolubles dans l'eau utilisés sont choisis dans le groupe constitué par phosphate, citrate, sulfate et carbonate de calcium, magnésium, fer, zinc et cuivre. On introduit l'un ou l'autre de ces sels selon le but désiré. La teneur en sels dans la suspension est comprise entre 0,1 et 10% en poids par rapport au poids total de la suspension.

En ce qui concerne la gomme de xanthane et la carboxyméthylcellulose, on l'introduit à raison de 0,1 à 1% en poids par rapport au poids total de la suspension.

Le traitement thermique de la suspension est effectué, soit de manière indirecte avec un système échangeur à plaques ou à tubes, soit de manière directe avec de la vapeur. Après ce traitement thermique, on laisse la suspension revenir à une température comprise entre 20 et 80° C. Elle est ainsi prête pour son utilisation, soit pour fabriquer du tofu, soit comme additif dans du lait ou des produits diététiques liquides stérilisés.

La matière de base pour la préparation du tofu ou fromage de soja est le lait de soja que l'on prépare de façon connue de la manière suivante : on lave à l'eau les graines entières de soja, puis on les immerge et on les fait tremper dans de l'eau pendant un temps prolongé. Le trempage des graines de soja dans de l'eau provoque leur gonflement. On effectue ensuite un broyage, une mouture ou un concassage desdites graines pour obtenir une purée ou pâte lisse, épaisse et blanche. Cette pâte ou purée est chauffée, puis filtrée et on obtient le lait de soja. Ce lait est stérilisé, puis mélangé avec 1 à 20% en poids de la suspension stérilisée précitée par rapport au poids total final et on effectue un remplissage aseptique à chaud ou à froid du mélange obtenu. La fonction du sel insoluble est d'induire la coagulation et la précipitation du tofu. On utilise de préférence comme sel du sulfate de calcium avec de la gomme de xanthane. Il est également possible d'ajouter dans la suspension stérilisée d'autres agents de coagulation, tels que $CaCl_2$, acide phytique et autres.

La suspension stérilisée, préparée selon l'invention, peut également servir d'additif de sels minéraux dans du lait ou des produits diététiques liquides stérilisés. Dans ce cas, on mélange 1 à 5% en poids de cette suspension par rapport au poids total final avec le lait ou des produits diététiques liquides stérilisés, et on effectue un remplissaage aseptique à froid ou à chaud du mélange obtenu. Par produits diététiques liquides on entend aussi bien des compositions d'alimentation par voie orale ou entérale.

Le type de sel utilisé dépend de la fonction que la boisson ainsi fabriquée est censée remplir. Il est bien entendu qu'on peut également ajouter des sels solubles à la suspension stabilisée.

La suite de la description est faite en référence aux exemples pour la préparation de tofu.

## Exemple 1

On soumet un lait de soja ayant une teneur totale en matières solides de 13% à un traitement Ultra-Haute-Température sur une ligne aseptique. Ce traitement thermique se fait à une température de 150° C pendant 2, 4 secondes. Le lait de soja est ensuite refroidi aseptiquement. On ajoute par ailleurs dans 95 l. d'eau 5 kg de $CaSO_4.2H_2O$ et

400 g de gomme de xanthane, vendu sous la marque Keltrol F par Kelco CO., à cette suspension. Ladite suspension est alors chauffée jusqu'à 135°C avec un chauffage à plaque et cette température est maintenue pendant 30 secondes. La suspension est ensuite refroidie aseptiquement jusqu'à 30°C. Le lait de soja stérilisé et la suspension de CaSO4 sont mélangés en continu avant le remplissage, de manière à obtenir un mélange contenant 92,5% de lait de soja et 7,5% de suspension de CaSO4. On passe alors sur la ligne de remplissage aseptique et on chaffe l'emballage final pour arriver à la coagulation du tofu dans le conteneur aseptique.

Exemple 2

Le lait de soja est traité par ultra haute température sur une ligne aseptique à 148°C pendant 5 secondes. Le produit quitte l'installation UHT à 85°C. On ajoute par ailleurs 6 kg de CaSO4.2H2O à 94 l. d'eau froide et 300 g de gomme de xanthane (Keltrol F) sont dissouts dans la suspension. Cette suspension stabilisée est chauffée à 140°C pendant 10 secondes, puis refroidie à 85°C.

Sur la machine de remplissage aseptique pour conteneurs de 200 ml, on introduit d'abord 16 ml de la suspension de CaSO4 et immédiatement après 184 ml de lait de soja chaud. La coagulation du tofu a lieu immédiatement et les conteneurs remplis doivent simplement être refroidis avant stockage.

On dispose ainsi d'un procédé permettant de préparer un tofu de longue conservation avec des agents de coagulation traditionnels.

## Revendications

1. Procédé de stérilisation d'une suspension aqueuse d'un sel ou de sels insolubles dans l'eau pour utilisation ultérieure dans un remplissage aseptique caractérisé en ce qu'on ajoute à ladite suspension un composé choisi dans le groupe constitué par la gomme de xanthane et la carboxyméthylcellulose et on la chauffe à une température comprise entre 120 et 150°C.

2. Procédé selon la revendication 1, caractérisé en ce que le sel insoluble dans l'eau utilisé est choisi dans le groupe constitué par phosphate, citrate, sulfate et carbonate de calcium, magnésium, fer, zinc et cuivre.

3. Procédé selon l'une des revendications 1 et 2, caractérisé en ce qu'on utilise entre 0,1 et 10% en poids de sels insolubles.

4. Procédé selon l'une des revendications 1 à 3, caractérisé en ce qu'on utilise entre 0,1 et 1% en poids de gomme de xanthane ou de carboxyméthylcellulose.

5. Utilisation de la suspension stérilisée obtenue selon l'une des revendications 1 à 4 pour la fabrication de tofu, caractérisé en ce qu'on mélange 1 à 20% en poids de cette suspension à du lait de soja stérilisé et on effectue en remplissage aseptique à chaud du mélange obtenu.

6. Utilisation de la suspension stérilisée obtenue selon l'une des revendications 1 à 4 pour l'addition de sels minéraux dans du lait ou des produits diététiques liquides stérilisés, caractérisé en ce qu'on mélange 1 à 5% en poids de cette suspension avec du lait ou des produits diététiques liquides stérilisés et on effectue un remplissage aseptique à froid ou à chaud du mélange obtenu.

## Claims

1. A process for sterilizing an aqueous suspension of a water-insoluble salt or salts for subsequent use in aseptic filling, characterized in that a compound selected from the group comprising xanthan gum and carboxymethyl cellulose is added to the suspension which is then heated to a temperature in the range from 120 to 150°C.

2. A process as claimed in claim 1, characterized in that the water-insoluble salt is selected from the group comprising calcium, magnesium, iron, zinc and copper phosphate, citrate, sulfate and carbonate.

3. A process as claimed in claim 1 or 2, characterized in that the water-insoluble salt(s) is/are used in a quantity of from 0.1 to 10% by weight.

4. A process as claimed in any of claims 1 to 3, characterized in that the xanthan gum or the carboxymethyl cellulose is used in a quantity of from 0.1 to 1% by weight.

5. The use of the sterilized suspension obtained by the process claimed in any of claims 1 to 4 for the production of tofu, characterized in that 1 to 20% by weight of the suspension is mixed with sterilized soya milk and the mixture obtained is aseptically filled while hot.

6. The use of the sterilized suspension obtained by the process claimed in any of claims 1 to 4 for the addition of mineral salts to milk or sterilized liquid dietetic products, characterized in that 1 to 5% by weight of the suspension is mixed with milk or sterilized liquid dietetic products and the mixture obtained is aseptically filled while hot or cold.

**Ansprüche**

1. Verfahren zum Sterilisieren einer wäßrigen Suspension eines wasserunlöslichen Salzes oder von wasserunlöslichen Salzen, welche Suspension für eine spätere Verwendung im Rahmen einer aseptischen Abfüllung bestimmt ist, dadurch gekennzeichnet, daß man zu dieser Suspension eine Verbindung zusetzt, welche aus der aus Xanthangummi und Carboxymethylcellulose bestehenden Gruppe ausgewählt ist, und daß man die Suspension auf eine zwischen 120 und 150° C liegende Temperatur erhitzt.

2. Verfahren nach Anspruch 1, dadurch gekennzeichnet, daß das verwendete wasserunlösliche Salz aus der aus Calcium-, Magnesium-, Eisen-, Zink- und Kupferphosphat, -citrat, -sulfat und -carbonat bestehenden Gruppe ausgewählt ist.

3. Verfahren nach Anspruch 1 oder 2, dadurch gekennzeichnet, daß man zwischen 0,1 und 10 Gew.-% an unlöslichen Salzen verwendet.

4. Verfahren nach einem der Ansprüche 1 bis 3, dadurch gekennzeichnet, daß man zwischen 0,1 und 1 Gew.-% an Xanthangummi oder Carboxymethylcellulose verwendet.

5. Verwendung der nach einem der Ansprüche 1 bis 4 erhaltenen sterilisierten Suspension für die Erzeugung von Tofu, dadurch gekennzeichnet, daß man 1 bis 20 Gew.-% dieser Suspension mit sterilisierter Sojamilch vermischt und daß man das so erhaltene Gemisch aseptisch heiß abfüllt.

6. Verwendung der nach einem der Ansprüche 1 bis 4 erhaltenen sterilisierten Suspension für einen Zusatz von Mineralsalzen zu sterilisierter Milch oder zu sterilisierten flüssigen Diätnahrungsmittelprodukten, dadurch gekennzeichnet, daß man 1 bis 5 Gew.-% dieser Suspension mit der sterilisierten Milch oder mit den sterilisierten flüssigen Nahrungsmittelprodukten vermischt und daß man das erhaltene Gemisch aseptisch kalt oder heiß abfüllt.